# EUROPEAN PATENT APPLICATION

(11) **EP 3 108 887 A1**
(43) Date of publication of application: **28.12.2016**
(21) Application number: 15752724.3
(22) Date of filing: 20.02.2015
(51) Int. Cl.: A61K 31/5575, A61K 9/08, A61P 27/02

(54) **PHARMACEUTICAL COMPOSITION FOR TREATING GEOGRAPHIC ATROPHY ASSOCIATED WITH AGE-RELATED MACULAR DEGENERATION**

(30) Priority: 21.02.2014 JP 2014032238; 11.09.2014 JP 2014185501
(71) Applicant: National University Corporation Kagawa University, Takamatsu-shi, Kagawa 760-8521 (JP); R-Tech Ueno, Ltd., Tokyo 100-0011 (JP)
(72) Inventor: SHIRAGA, Fumio, Okayama-shi Okayama 703-8281 (JP); SHIRAGAMI, Chieko, Kita-gun Kagawa 761-0793 (JP); MASHIMA, Yukihiko, Tokyo 100-0011 (JP)
(74) Representative: Adam, Holger
(86) International application number: PCT/JP2015/054842
(87) International publication number: WO 2015/125933

(57) **Abstract**

Disclosed is a pharmaceutical composition for use in the treatment of geographic atrophy associated with age-related macular degeneration in a subject, which comprises administering a 15-keto-prostaglandin compound to the subject in need of the treatment. The 15-keto-prostaglandin compound such as Isopropyl Unoprostone is useful for treating geographic atrophy as well as for preventing vision loss caused by geographic atrophy.

## Description

### TECHNICAL FIELD

The present invention relates to a composition for treating geographic atrophy (GA) in a patient with age-related macular degeneration (AMD).

### BACKGROUND ART

Age-related macular degeneration (AMD) is a condition that progresses with age and causes damage to the macula, a small spot near the center of the retina. Macula is the part most seriously responsible for the visual acuity and AMD is currently the fourth leading cause of vision loss in Japan. There are two types of AMD, known as "exudative AMD" and "dry AMD". Exudative AMD is a disease in which choroidal neovascularization occurs underneath the macula in the retina and causes metamorphopsia, central scotoma, and severe vision loss. In these days, patients with exudative AMD are treated by photodynamic therapy (PDT), intravitreal injection of an inhibitor of vascular endothelial growth factor or combination of PDT and IVR.

In patients who had exudative AMD and had been treated by the conventional treatment, deterioration of the retinal function in and around the macula, declining of the eyesight and enlargement of the central scotoma may be observed after the exudative lesion had disappeared by the conventional treatment (Non-Patent Literature 1). In such cases as above, many patients have geographic atrophy (GA) in the macula and in the region surrounding the macula. GA has been thought to be an important indicator of poor visual prognosis in eyes with AMD (Non-Patent Literatures 1-3). For example, GA occurred in 80% of patients with retinal angiomarous proliferation, one variant of exudative AMD (Non-Patent Literature 4). GA occurred in 35-45% of patients with AMD (Non-Patent Literature 5).

There are various way to diagnose GA. For example, funds autofluorescent (FAF) images may be analyzed by RegionFinder^{™} software (Heidelberg Engineering, Germany) to determine the presence of GA and its size precisely (Non-Patent Literature 8).

The cause of GA has not yet been revealed and no established treatment for GA is available. Various treatment have been proposed but the effects of them have been unknown (Non-Patent Literature 9). Many times, GA has been discussed in relation to dry AMD. However, the condition also relates to exudative AMD. There are great desire for a method that can effectively treat GA irrespective of the underlining AMD, exudative or dry.

Prostaglandins (hereinafter, referred to as PG(s)) are members of class of organic carboxylic acids, which are contained in tissues or organs of human or other mammals, and exhibit a wide range of physiological activities. PGs found in nature (primary PGs) have, as a general structural property thereof, a prostanoic acid skeleton as shown in formula (A):

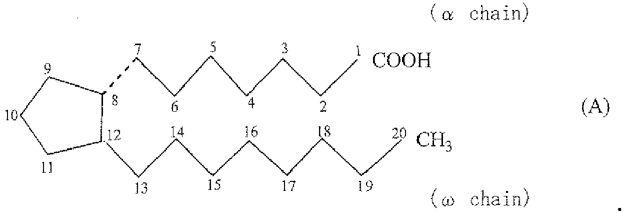

On the other hand, some of synthetic analogues of primary PGs have modified skeletons. The primary PGs are classified to FGAs, PGBs, PGCs, PGDs, PGEs, PGFs, PGGs, PGHs, PGIs and PGJs according to the structure of the five-membered ring moiety, and further classified into the following three types by the number and position of the unsaturated bond at the carbon chain moiety:
Subscript 1: 13,14-unsaturated-15-OH
Subscript 2: 5,6- and 13,14-diunsaturated-15-OH
Subscript 3: 5,6-, 13,14-, and 17,18-triunsaturated-15-OH.

Further, the PGFs are classified according to the configuration of the hydroxyl group at the 9-position, into α type (the hydroxyl group is of an α-configuration) and β type (the hydroxyl group is of a β-configuration).

Furthermore, some of 15-keto PGs, which have an oxo group at position 15 of PGs in place of the hydroxy group, and 13,14-dihydro-15-keto-PGs, which have a single bond between positions 13 and 14 of PGs, are known as substances naturally produced by enzymatic actions during in vivo metabolism of primary PGs and have some therapeutic effect. For example, 15-keto-prostaglandin compounds have been known to be useful for the treatment of ocular hypertension and glaucoma (USP Nos. 5,001,153 and 5,151,444, these publications are incorporated herein by reference).

### [Patent Literature]

[Patent Literature 1] US Patent No. 5,001,153
[Patent Literature 2] US Patent No. 5,151,444
[Patent Literature 3] JP Laid Open Application No. 2012-524025

### [Non-Patent Literature]

[Non-Patent Literature 1] Potter JW, et al., J Am Optom Assoc. 1981, 52, 503-508
[Non-Patent Literature 2] Sunness JS, et al., Ophthalmology 1999, 106, 1768-1779
[Non-Patent Literature 3] Schatz H and McDonald HR., Ophthalmology 1989, 96, 1541-1551
[Non-Patent Literature 4] McBain VA, et al., Retina 2011, 31, 1043-1052
[Non-Patent Literature 5] Smith W, et al., Ophthalmology 2001, 108, 697-704
[Non-Patent Literature 6] Sunness Js, et al., Ophthalmology 2007, 114, 271-277
[Non-Patent Literature 7] Holz FG and FAM-Study Group, Am. J. Ophthalmol. 2007, 143, 463-72
[Non-Patent Literature 8] Schmitz-Valckenberg S, et al., Invest. Ophthalmol. Vis. Sci. 2011, 52, 7640-7646
[Non-Patent Literature 9] Juan E. Grunwald et al., Ophthalmol. 2014, 121, 150-161
[Non-Patent Literature 10] Yamamoto S. et al., Ophthalmol. Ther. 2012, 1, 5

### SUMMARY OF THE INVENTION

### TECHNICAL PROBLEM

An object of the present invention is to provide a method and pharmaceutical composition for treating geographic atrophy associated with age-related macular degeneration in a mammalian subject including human.

### SOLUTION TO PROBLEM

In one embodiment, provided is a pharmaceutical composition for use in the treatment of GA associated with AMD, comprising a 15-keto-prostaglandin compound.

In another embodiment, provided is a method for treating GA associated with AMD in a subject in need thereof, which comprises administering to the subject an effective amount of a 15-keto-prostaglandin compound.

In a further embodiment, provided is a use of 15-keto-prostaglandin compound for the manufacture of a pharmaceutical product for treating GA associated with AMD.

### ADVANTAGEOUS EFFECTS OF INVENTION

The pharmaceutical composition as described herein is useful for treating GA associated with AMD and also for preventing vision loss caused by GA associated with AMD.

### BRIEF EXPLANATION OF DRAWINGS

Figure 1-A is a fundus image of a patient in the U group before receiving the treatment.
Figure 1-B is a fundus autofluorescence image of a patient in the U group.
Figure 1-C is an optical coherence tomographic image of a patient in the U group.
Figure 2 shows fundus autofluorescence images and the time-dependent change in GA area of a patient in the U group.
Figure 3-A is a fundus photograph of a patient in the P group before receiving the treatment.
Figure 3-B is a fundus autofluorescence image of a patient in the P group.
Figure 3-C is an optical coherence tomographic image of a patient in the P group.
Figure 4 shows fundus autofluorescence images and the time-dependent change in GA area of a patient in the P group.
Figure 5 is a graph showing the time-dependent change in GA area.
Figure 6 is a graph showing the time-dependent change in GA. enlargement ratio.
Figure 7 is a graph showing the time-dependent change in logMAR visual acuity.
Figure 8 is a graph showing the relation between change in GA area and the AMD subtypes at week 54.

### DESCRIPTION OF EMBODIMENTS

In the specification and claims, "15-keto-prostaglandin compound" (hereinafter, referred to as "15-keto-PG compound") may include a compound that has the prostanoic acid skeleton and an oxo group at its position 15 instead of the hydroxy group; derivatives, including substituted derivatives; or analogs of the compound, irrespective of the configuration of the five-membered ring, the number of double bonds, presence or absence of a substituent, or any other modification in the α or ω chain.

The nomenclature of the 15-keto-PG compounds used herein is based on the numbering system of the prostanoic acid represented in the above formula (A).

The formula (A) shows a basic skeleton of the C-20 carbon atoms, but the 15-keto-PG compounds in the present invention are not limited to those having the same number of carbon atoms. In the formula (A), the numbering of the carbon atoms which constitute the basic skeleton of the PG compounds starts at the carboxylic acid (numbered 1), and carbon atoms in the α-chain are numbered 2 to 7 towards the five-membered ring, those in the ring are 8 to 12, and those in the α-chain are 13 to 20. When the number of carbon atoms is decreased in the α-chain, the number is deleted in the order starting from position 2; and when the number of carbon atoms is increased in the α-chain, compounds are named as substitution compounds having respective substituents at position 2 in place of the carboxy group (C-1). Similarly, when the number of carbon atoms is decreased in the ω-chain, the number is deleted in the order starting from position 20; and when the number of carbon atoms is increased in the ω-chain, the carbon atoms beyound position 20 are named as substituents. Stereochemistry of the compounds is the same as that of the above formula (A) unless otherwise specified.

In general, each of the terms PGD, PGE and PGF represents a PG compound having hydroxy groups at positions 9 and/or 11, but in the present specification, these terms also include those having substituents other than the hydroxy group at positions 9 and/or 11. Such compounds are referred to as 9-dehydroxy-9-substituted-PG compounds or 11-dehydroxy-11-substituted-PG compounds. A PG compound having hydrogen in place of the hydroxy group is simply named as 9- or 11-deoxy-PG compound.

As stated above, the nomenclature of the 15-keto-prostaglandin compounds is based on the prostanoic acid skeleton. However, in case the compound has a similar partial construction as a prostaglandin, the abbreviation of "PG" may be used. Thus, a PG compound of which α-chain is extended by two carbon atoms, that is, having 9 carbon atoms in the α-chain is named as 2-decarboxy-2-(2-carboxyethyl)-15-keto-PG compound. Similarly, a PG compound having 11 carbon atoms in the α-chain is named as 2-decarboxy-2-(4-carboxybutyl)-15-keto-PGF compound. Further, a PG compound of which ω-chain is extended by two carbon atoms, that is, having 10 carbon atoms in the ω-chain is named as 15-keto-20-ethyl-PG compound. These compounds, however, may also be named according to the IUPAC nomenclatures.

The 15-keto-PGs used herein may include any PG derivatives or analogs insofar as having an oxo group at position 15 in place of the hydroxy group. Accordingly, for example, a 15-keto-PG type 1 compound having a double bond at 13-14 position, a 15-keto-PG type 2 compound having two double bond at 13-14 and 5-6 position, a 15-keto-PG type 3 compound having three double bond at 5-6, 13-14 and 17-18 position, 13,14-dihydro-15-keto-PG compound wherein the double bond at 13-14 position is single bond.

Typical examples of the compounds used herein include 15-keto-PG type 1, 15-keto-PG type 2, 15-keto- PG type 3, 13, 14-dihydro-15-keto-PG type 1, 13,14-dihydro-15-keto-PG type 2, 13, 14-dihydro-15-keto-PG type 3 and the derivatives or analogs thereof.

Examples of the analogs (including substituted derivatives) or derivatives include a 15-keto-PG compound of which carboxy group at the end of α-chain is esterified; a compound of which α-chain is extended; physiologically acceptable salt thereof; a compound having a double bond at 2-3 position or a triple bond at position 5-6, a compound having substituent (s) at position 3, 5, 6, 16, 17, 18, 19 and/or 20; and a compound having lower alkyl or a hydroxy (lower) alkyl group at position 9 and/or 11 in place of the hydroxy group.

According to the present invention, preferred substituents at position 3, 17, 18 and/or 19 include alkyl having 1-4 carbon atoms, especially methyl and ethyl. Preferred substituents at position 16 include lower alkyl such as methyl and ethyl, hydroxy, halogen atoms such as chlorine and fluorine, and aryloxy such as trifluoromethylphenoxy. Preferred substituents at position 17 include lower alkyl such as methyl and ethyl, hydroxy, halogen atoms such as chlorine and fluorine, aryloxy such as trifluoromethylphenoxy. Preferred substituents at position 20 include saturated or unsaturated lower alkyl such as C1-4 alkyl, lower alkoxy such as C1-4 alkoxy, and lower alkoxy alkyl such as C1-4 alkoxy-C1-4 alkyl. Preferred substuents at position 5 include halogen atoms such as chlorine and fluorine. Preferred substituents at position 6 include an oxo group forming a carboxyl group. Stereochemistry of PGs having hydroxy, lower alkyl or hydroxy(lower)alkyl substituent at position 9 and 11 may be α, βor a mixture thereof.

Further, the above analogs may be compounds having an alkoxy, cycloalkyl, cycloalkyloxy, phenoxy or phenyl group at the end of the ω-chain where the chain is shorter than the primary PGs.

Especially preferred compounds include a 13,14-dihydro-15-keto-PG compound which has a single bond between positions 13-14; a compound of which ω-chain is extended.

A preferred compounds used in the present invention is represented by the formula (I): 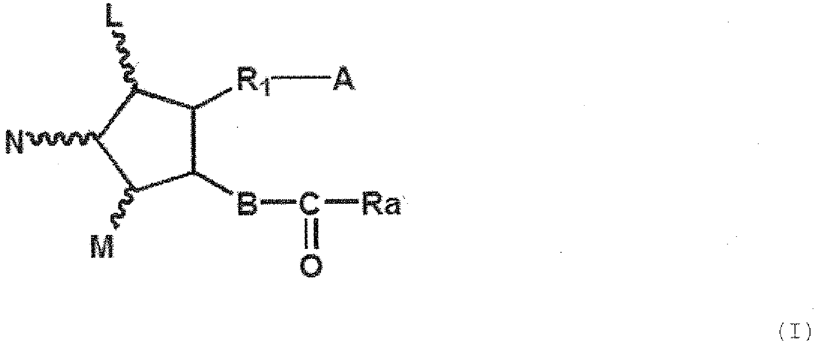
wherein L, M and N are hydrogen, hydroxy, halogen, lower alkyl, hydroxy (lower) alkyl, or oxo, and the five-membered ring may have at least one double bond;
A is -CH₃, -CH₂OH, -COCH₂OH, -COOH or a functional derivative thereof;
B is -CH₂-CH₂-, -CH=CH- or -C≡C-;
R₁ is a saturated or unsaturated bivalent lower or medium aliphatic hydrocarbon residue, which is unsubstituted or substituted with halogen, lower alkyl hydroxy, oxo, aryl or heterocyclic group, and at least one of carbon atom in the aliphatic hydrocarbon is optionally substituted by oxygen, nitrogen or sulfur; and
Ra is a saturated or unsaturated lower or medium aliphatic hydrocarbon group, which is unsubstituted or substituted with halogen, oxo, hydroxy, lower alkyl lower alkoxy, lower alkanoyloxy, cyclo(lower)alkyl, cyclo(lower)alkyloxy, aryl, aryloxy, heterocyclic group or hetrocyclic-oxy group; lower alkoxy, lower aklanoyloxy, cyclo(lower)alkyl; cycle(lower)alkyloxy; aryl; aryloxy,; heterocyclic group; heterocyclic-oxy group.

A group of particularly preferable compounds among the above described compounds is represented by the formula (II): 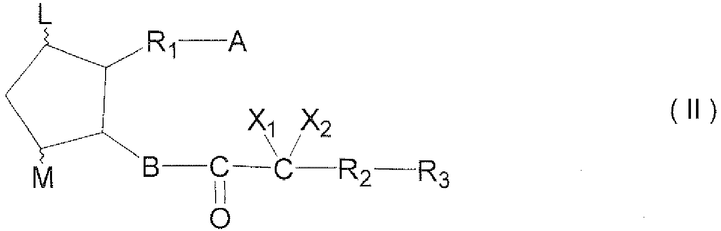
wherein L and M are hydrogen, hydroxy, halogen, lower alkyl hydroxy (lower) alkyl lower alkanoyloxy or oxo, and the five-membered ring may have at least one double bond;
A is -CH₃, -CH₂OH, -COCH₂OH, -COOH or a functional derivative thereof;
B is -CH₂-CH₂-, -CH=CH-, -C≡C-;
X1, and X₂ are hydrogen, lower alkyl, or halogen;
R₁ is a saturated or unsaturated bivalent lower or medium aliphatic hydrocarbon residue, which is unsubstituted or substituted with halogen, lower alkyl hydroxy, oxo, aryl or heterocyclic group, and at least one of carbon atom in the aliphatic hydrocarbon is optionally substituted by oxygen, nitrogen or sulfur;
R₂ is a single bond or lower alkylene; and
R₃ is lower alkyl lower alkoxy, lower alkanoyloxy, cyclo (lower) alkyl, cyclo(lower)alkyloxy, aryl, aryloxy, heterocyclic group or heterocyclic-oxy group.

In the above formula, the term "unsaturated" in the definitions for R₁ and Ra is intended to include at least one or more double bonds and/or triple bonds that are individually, separately or serially present between carbon atoms of the main and/or side chains. According to the usual nomenclature, an unsaturated bond between two serial positions is represented by denoting the lower number of the two positions, and an unsaturated bond between two distal positions is represented by denoting both of the positions.

The term "lower or medium aliphatic hydrocarbon" refers to a straight or branched chain hydrocarbon group having 1 to 14 carbon atoms (for a side chain, 1 to 3 carbon atoms are preferable) and preferably 1 to 10, especially 1 to 8 carbon atoms.

The term "halogen atom" covers fluorine, chlorine, bromine and iodine.

The term "lower" throughout the specification is intended to include a group having 1 to 6 carbon atoms unless otherwise specified.

The term "lower alkyl" refers to a straight or branched chain saturated hydrocarbon group containing 1 to 6 carbon atoms and includes, for example, ethyl ethyl, propyl, isopropyl, butyl, isobutyl, t-butyl, pentyl and hexyl.

The term "lower alkylene" refers to a straight or branched chain bivalent saturated hydrocarbon group containing 1 to 6 carbon atoms and includes, for example, methylene, ethylene, propylene, isopropylene, butylene, isobutylene, t-butylene, pentylene and hexylene.

The term "lower alkoxy" refers to a group of lower alkyl-O-, wherein lower alkyl is as defined above.

The term "hydroxy(lower)alkyl" refers to a lower alkyl as defined above which is substituted with at least one hydroxy group such as hydroxymethyl, 1-hydroxyethyl, 2-hydroxyethyl and 1-methyl-1-hydroxyethyl.

The term "lower alkanoyloxy" refers to a group represented by the formula RCO-O-, wherein RCO- is an acyl group formed by oxidation of a lower alkyl group as defined above, such as acetyl.

The term "cycle(lower)alkyl" refers to a cyclic group formed by cyclization of a lower alkyl group as defined above but contains three or more carbon atoms, and includes, for example, cyclopropyl, cyclobutyl, cyclopentyl and cyclohexyl.

The term "cycle(lower)alkyloxy" refers to the group of cycle(lower)alkyl-O-, wherein cyclo(lower)alkyl is as defined above.

The term "aryl" may include unsubstituted or substituted aromatic hydrocarbon rings (preferably monocyclic groups), for example, phenyl, tolyl, xylyl. Examples of the substituents are halogen atom and halo (lower) alkyl, wherein halogen atom and lower alkyl are as defined above.

The term "aryloxy" refers to a group represented by the formula ArO-, wherein Ar is aryl as defined above.

The term "heterocyclic group" may include mono-to tri-cyclic, preferably monocyclic heterocyclic group which is 5 to 14, preferably 5 to 10 membered ring having optionally substituted carbon atom and 1 to 4, preferably 1 to 3 of 1 or 2 type of hetero atoms selected from nitrogen atom, oxygen atom and sulfur atom. Examples of the heterocyclic group include furyl, thienyl, pyrrolyl, oxazolyl, isoxazolyl, thiazolyl, isothiazolyl, imidazolyl, pyrazolyl, furazanyl, pyranyl, pyridyl, pyridazinyl, pyrimidyl, pyrazinyl, 2-pyrrolinyl, pyrrolidinyl, 2-imidazolinyl, imidazolidinyl, 2-pyrazolinyl, pyrazolidinyl, piperidino, piperazinyl, morpholino, indolyl, benzothienyl, quinolyl, isoquinolyl, purinyl, quinazolinyl, carbazolyl, acridinyl, phenanthridinyl, benzimidazolyl, benzimidazolinyl, benzothiazolyl, phenothiazinyl. Examples of the substituent in this case include halogen, and halogen substituted lower alkyl group, wherein halogen atom and lower alkyl group are as described above.

The term "heterocyclic-oxy group" means a group represented by the formula HcO-, wherein Hc is a heterocyclic group as described above.

The term "functional derivative" of A includes salts (preferably pharmaceutically acceptable salts), ethers, esters and amides.

Suitable "pharmaceutically acceptable salts" include conventionally used non-toxic salts, for example a salt with an inorganic base such as an alkali metal salt (such as sodium salt and potassium salt), an alkaline earth metal salt (such as calcium salt and magnesium salt), an ammonium salt; or a salt with an organic base, for example, an amine salt (such as methylamine salt, dimethylamine salt, cyclohexylamine salt, benzylamine salt, piperidine salt, ethylenediamine salt, ethanolamine salt, diethanolamine salt, triethanolamine salt, tris (hydroxymethylamino) ethane salt, monomethyl- monoethanolamine salt, procaine salt and caffeine salt), a basic amino acid salt (such as arginine salt and lysine salt), tetraalkyl ammonium salt and the like. These salts may be prepared by a conventional process, for example from the corresponding acid and base or by salt interchange.

Examples of the ethers include alkyl ethers, for example, lower alkyl ethers such as methyl ether, ethyl ether, propyl ether, isopropyl ether, butyl ether, isobutyl ether, t-butyl ether, pentyl ether and 1-cyclopropyl ethyl ether; and medium or higher alkyl ethers such as octyl ether, diethylhexyl ether, lauryl ether and cetyl ether; unsaturated ethers such as oleyl ether and linolenyl ether; lower alkenyl ethers such as vinyl ether, allyl ether; lower alkenyl ethers such as ethynyl ether and propynyl ether; hydroxy (lower) alkyl ethers such as hydroxyethyl ether and hydroxyisopropyl ether; lower alkoxy (lower) alkyl ethers such as methoxymethyl ether and 1-metroxyethyl ether; optionally substituted aryl ethers such as phenyl ether, tosyl ether, t-butylphenyl ether, salicyl ether, 3,4-di-methoxyphenyl ether and benzamidophenyl ether; and aryl(lower)alkyl ethers such as benzyl ether, trityl ether and benzhydryl ether.

Examples of the esters include aliphatic esters, for example, lower alkyl esters such as methyl ester, ethyl ester, propyl ester, isopropyl ester, butyl ester, isobutyl ester, t-butyl ester, pentyl ester and 1-cyclopropylethyl ester; lower alkenyl esters such as vinyl ester and allyl ester; lower alkynyl esters such as ethynyl ester and propynyl ester; hydroxy(lower)alkyl ester such as hydroxyethyl ester; lower alkoxy (lower) alkyl esters such as methoxymethyl ester and 1-methoxyethyl ester; and optionally substituted aryl esters such as, for example, phenyl ester, tolyl ester, t-butylphenyl ester, salicyl ester, 3, 4-di-methoxyphenyl ester and benzamidophenyl ester; and aryl (lower) alkyl ester such as benzyl ester, trityl ester and benzhydryl ester.

The amide of A mean a group represented by the formula -CONR'R", wherein each of R' and R" is hydrogen, lower alkyl aryl, alkyl- or aryl-sulfonyl, lower alkenyl and lower alkynyl, and include for example lower alkyl amides such as methylamide, ethylamide, dimethylamide and diethylamide; arylamides such as anilide and toluidide; and alkyl- or aryl-sulfonylamides such as methylsulfonylamide, ethylsulfonyl-amide and tolylsulfonylamide.

Preferred examples of L and M are a combination wherein both of them are hydroxy which provides a 5-membered ring structure of, so called, PGF type; a combination wherein L is hydroxy and M is oxo which provides a 5-membered ring structure of, so called, PGE type, and a combination wherein L is oxo and M is hydrogen which provides a 5-membered ring structure of, so called, 11-deoxy-PG type.

Preferred example A is -COOH, its pharmaceutically acceptable salt, ester or amide thereof.

Preferred example B is -CH₂-CH₂-, which provides the structure of so-called, 13,14-dihydro type.

Preferred example of X₁ and X₂ are hydrogen or halogen, preferably at least one of them is halogen, more preferably, both of them are halogen, especially, fluorine that provides a structure of, so called 16,16-difluoro type.

Preferred R₁ is a hydrocarbon containing 1-10 carbon atoms, preferably, 6-10 carbon atoms. Further, at least one carbon atom in the aliphatic hydrocarbon is optionally substituted by oxygen, nitrogen or sulfur.

Examples of R₁ include, for example, the following groups:

-CH₂-CH₂-CH₂-CH₂-CH₂-CH₂-,

-CH₂-CH=CH-CH₂-CH₂-CH₂-,

-CH₂-CH₂-CH₂-CH₂-CH=CH-,

-CH₂-C=C≡CH₂-CH₂-CH₂-,

-CH₂-CH₂-CH₂ -CH₂-O-CH₂-,

-CH₂-CH=CH-CH₂-O-CH₂-,

-CH₂-C≡C-CH₂-O-CH₂-,

-CH₂-CH₂-CH₂-CH₂ -CH₂ -CH₂ -CH₂-,

-CH₂-CH=CH-CH₂-CH₂ -CH₂-CH₂-,

-CH₂-CH₂-CH₂-CH₂-CH₂-CH=CH-,

-CH₂-C≡C-CH₂-CH₂-CHz-CH₂-,

-CH₂-CH₂-CH₂-CH₂-CH₂-CH (CH₃)-CH₂-,

-CH₂-CH₂-CH₂-CH₂-CH(CH₃)-CH₂-,

-CH₂-CH₂-CH₂-CH₂-CH₂-CH₂-CH₂-CH₂-,

-CH₂-CH=CH-CH₂-CH₂-CH₂-CH₂-CH₂-,

-CH₂-CH₂-CH₂-CH₂-CH₂-CH₂-CH=CH-,

-CH₂-C≡C-CH₂-CH₂-CH₂-CH₂-CH₂-, and

-CH₂-CH₂-CH₂-CH₂-CH₂-CH₂-CH (CH₃) -CH₂-.

Preferred Ra is a hydrocarbon containing 1-10 carbon atoms, more preferably, 1-8 carbon atoms. Ra may have one or two side chains having one carbon atom.

The configuration of the ring and the α- and/or ω chains in the above formula (I) and (II) may be the same as or different from that of the primary PGs. However, the present invention also includes a mixture of a compound having a primary type configuration and a compound of a non-primary type configuration.

The Examples of the typical compound in the invention is 13,14-dihydro-15-keto-20-ethyl PGF compound, and the derivatives or analogs thereof. The example of most preferable compound in the invention is 13,14-dihydro-15-keto-20-ethyl F_{2 _{α}} isopropyl ester (hereinafter, it is also referred to as "isopropyl unoprostone").

In the present invention, the PG compound which is dihydro between 13 and 14, and keto (=O) at 15 position may be in the keto-hemiacetal equilibrium by formation of a hemiacetal between hydroxy at position 11 and keto at position 15.

For example, it has been revealed that when both of X₁ and X₂ are halogen atoms, especially, fluorine atoms, the compound contains a tautomeric isomer, bicyclic compound.

If such tautomeric isomers as above are present, the proportion of both tautomeric isomers varies with the structure of the rest of the molecule or the kind of the substituent present. Sometimes one isomer may predominantly be present in comparison with the other. However, it is to be appreciated that the present invention includes both isomers.

Further, the 15-keto-PG compounds used in the invention include the bicyclic compound and analogs or derivatives thereof.

The bicyclic compound is represented by the formula (III): 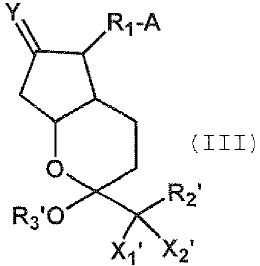
wherein, A is -CH₃, or -CH₂OH -COCH₂OH -COOH or a functional derivative thereof;
K₁ 'and X₂' are hydrogen, lower alkyl or halogen;
Y is 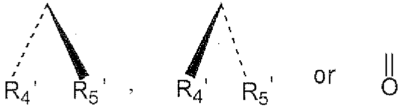
   wherein R₄ 'and R₅' are hydrogen, hydroxy, halogen, lower alkyl, lower alkoxy or hydroxy (lower) alkyl, wherein R₄' and R₅' are not hydroxy and lower alkoxy at the same time.
R₁ is a saturated or unsaturated divalent lower or medium aliphatic hydrocarbon residue, which is unsubstituted or substituted with halogen, alkyl, hydroxy, oxo, aryl or heterocyclic group, and at least one of carbon atom in the aliphatic hydrocarbon is optionally substituted by oxygen, nitrogen or sulfur; and
R₂' is a saturated or unsaturated lower or medium aliphatic hydrocarbon residue, which is unsubstituted or substituted with halogen, oxo, hydroxy, lower alkyl, lower alkoxy, lower alkanoyloxy, cycle (lower) alkyl, cyclo (lower) alkyloxy, aryl, aryloxy, heterocyclic group or hetrocyclic-oxy group; lower alkoxy; lower alkanoyloxy; cyclo (lower) alkyl; cyclo (lower) alkyloxy; aryl; aryloxy; heterocyclic group; heterocyclic-oxy group.
R₃' is hydrogen, lower alkyl, cyclo(lower)alkyl, aryl or heterocyclic group.

Furthermore, while the compounds used in the invention may be represented by a formula or name based on keto-type regardless of the presence or absence of the isomers, it is to be noted that such structure or name does not intend to exclude the hemiacetal type compound.

In the present invention, any of isomers such as the individual tautomeric isomers, the mixture thereof, or optical isomers, the mixture thereof, a racemic mixture, and other steric isomers may be used in the same purpose.

Some of the compounds used in the present invention may be prepared by the method disclosed in USP Nos. 5,073,569, 5,166,174, 5, 221, 763, 5,212,324, 5,739,161 and 6,242,485 (these cited references are herein incorporated by reference).

The present invention provides a pharmaceutical composition and a method for treating geographic atrophy (GA) associated with age-related macular degeneration (AMD). The subject to be treated by the composition or method of the present invention is a mammalian subject including human.

The term "treat" or "treatment" used herein includes any means of control such as prevention, care, relief of the condition, attenuation of the condition or disease and arrest of progression of the disease.

In the specification and claims of this application, the term "AMD" represents all types of AMD including dry AMD and exudative AMD, unless otherwise indicated.

The expression "GA associated with AMD" includes GA observed in a subject who is currently suffered from AMD and also GA observed in a subject who had received the treatment for AMD. For example, GA observed after the exudative lesions have disappeared by a treatment for exudative AMD in a subject who had exudative AMD is included.

The subject to be treated by the pharmaceutical composition of the present invention may include a subject who is diagnosed with GA and also a subject who is likely to develop GA. To the subject who is likely to develop GA, the treatment with the pharmaceutical composition may be given together with the conventional treatment for AMD. For example, a patient with exudative AMD may be given with a conventional treatment for AMD such as IVR or PDT in combination with the treatment with the pharmaceutical composition of the present invention irrespective of the presence or absence of GA. Alternatively, a patient with exudative AMD may be treated by a conventional treatment and after the exudative lesion disappears, the patient may be treated by the pharmaceutical composition of the present invention irrespective of the presence or absence of GA.

According to the present invention, the pharmaceutical composition may be administered systemically or topically. Usually, the compound may be administered by oral administration, intravenous injection (including infusion), ocular topical administration (e.g. periocular (e.g., subTenon's), subconjunctival, intraocular, intravitreal, intracameral, subretinal, suprachoroidal, and retrobulbar administrations) and the like.

The dose may vary depending on the strain of the animal, age, body weight, symptom to be treated, desired therapeutic effect, administration route, term of treatment and the like. A satisfactory effect may be obtained by systemic administration 1-4 times per day or continuous administration at the amount of 0.00001-500mg/kg per day, and preferably 0.0001-100mg/kg per day.

The compound may preferably be formulated in a pharmaceutical composition suitable for administration in a conventional manner. The composition may be those suitable for oral administration, ocular topical administration, injection or perfusion as well as it may be an external agent.

The composition of the present invention may further contain physiologically acceptable additives. Said additives may include the ingredients used with the compound of the present invention such as an excipient, diluent, filler, resolvent, lubricant, adjuvant, binder, disintegrator, coating agent, capsulating agent, ointment base, suppository base, aerozoling agent, emulsifier, dispersing agent, suspending agent, thickener, tonicity agent, buffering agent, soothing agent, preservative, antioxidant, corrigent, flavor, colorant, a functional material such as cyclodextrin and biodegradable polymer, and stabilizer. The additives are well known to the art and may be selected from those generally described in reference books of pharmaceutics.

The amount of the above-defined compound in the composition of the invention may vary depending on the formulation of the composition, and may generally be 0.000001-10.0%, more preferably 0.00001-5.0%, and most preferably 0.0001-1%.

Examples of solid compositions for oral administration include tablets, troches, sublingual tablets, capsules, pills, powders, granules and the like. The solid composition may be prepared by mixing one or more active ingredients with at least one inactive diluent. The composition may further contain additives other than the inactive diluents, for example, a lubricant, a disintegrator and a stabilizer. Tablets and pills may be coated with an enteric or gastroenteric film, if necessary.

The composition may be covered with two or more layers. The composition may also be adsorbed to a sustained release material, or filled in microcapsules. Additionally, the composition may be filled in capsules made of an easily degradable material such as gelatin. The composition may be dissolved in an appropriate solvent such as fatty acid or its mono, di or triglyceride and filled in a soft capsule. Sublingual tablet may be used in need of fast-acting property.

Examples of liquid compositions for oral administration include emulsions, solutions, suspensions, syrups and elixirs and the like. Said composition may further contain a conventionally used inactive diluents e.g. Purified water or ethyl alcohol. The composition may contain additives other than the inactive diluents such as adjuvant e.g. wetting agents and suspending agents, sweeteners, flavors, fragrance and preservatives.

The composition of the present invention may be in the form of spraying composition, which contains one or more active ingredients and may be prepared according to a known method.

Examples of injectable compositions of the present invention for parenteral administration include sterile aqueous or non-aqueous solutions, suspensions and emulsions.

Diluents for the aqueous solution or suspension may include, for example, distilled water for injection, physiological saline and Ringer's solution.

Non-aqueous diluents for solution and suspension may include, for example, propylene glycol, polyethylene glycol, vegetable oils such as olive oil, alcohols such as ethanol and polysorbate. The composition may further comprise additives such as preservatives, wetting agents, emulsifying agents, dispersing agents and the like. They may be sterilized by filtration through, e.g. a bacteria-retaining filter, compounding with a sterilizer, or by means of gas or radioisotope irradiation sterilization.

The injectable composition may also be provided as a sterilized powder composition to be dissolved in a sterilized solvent for injection before use.

The composition of the present invention may also be an ophthalmic formulation such as eye drops and eye ointments. Examples of ophthalmic formulations may include all ophthalmic formulations for topical ocular administration used in the ophthalmic field.

The eye drops are prepared by dissolving the active ingredient in a sterile aqueous solution such as saline and buffering solution. The eye drops may be provided as a powder composition to be dissolved before use, or as a combination of powder compositions to be dissolved before use. The eye ointments are prepared by mixing the active ingredient into an ointment base. The formulations are prepared according to the conventional methods.

Osmolarity modifiers include sodium chloride, potassium chloride, calcium chloride, sodium bicarbonate, sodium carbonate, magnesium sulfate, sodium hydrogen phosphate, sodium dihydrogen phosphate, dipotassium hydrogen phosphate, boric acid, borax, sodium hydroxide, hydrochloric acid, mannitol, isosorbitol, propylene glycol, glucose and glycerine, but not limited thereto, as far as they are ordinarily used in the ophthalmic field.

Further, additives ordinarily used in the ophthalmic field may be added to the present composition as desired. Such additives as above include, for example, buffer agent (e.g., boric acid, sodium monohydrogen phosphate and sodium dihydrogen phosphate), preservatives (e.g., benzalkonium chloride, benzethonium chloride and chlorobutanol), thickeners (e.g., saccharide such as lactose, mannitol and maltose; e.g., hyaluronic acid or its salt such as sodium hyaluronate and potassium hyaluronate; e.g., mucopolysaccharide such as chondroitin sulfate; e.g., sodium polyacrylate, carboxyvinyl polymer and crosslinked polyacrylate).

When the composition of the present invention is provided as eye ointment, the composition may contain ordinarily used eye ointment base in addition to the additives discussed above. Examples of the eye ointment base include, but not limited to, oil base such as vaseline, liquid paraffin, polyethylene, selen 50, plastibase, macrogol or a combination thereof; emulsion base having oil phase and water phase emulsified with surfactant; and water soluble base such as hydroxypropylmethylcellulose, carboxypropylmethylcellulose and polyethylene glycol.

In one preferred embodiment of the present invention, the ophthalmic composition contains substantially no benzalkonium chloride. The expression "the ophthalmic composition contains substantially no benzalkonium. chloride" used herein means that the composition contains no benzalkonium chloride, or the composition contains benzalkonium chloride as low as possible. In the present invention, the "ophthalmic composition containing substantially no benzalkonium chloride" may contain Benzalkonium chloride at a concentration of 0.01% or less, preferably 5-6, or less, more preferably 0.003% or less.

The eye drops of the present invention may be a sterile unit dose type formulation (one day type or single unit dose type) containing no preservatives such as benzalkonium chloride.

The ophthalmic composition may further include sustained release forms such as gel formulation, liposome formulation, lipid microemulsion formulation, microsphere formulation, nanosphere formulation and implant formulation in order to provide the active compound sustainedly to the back of the eye.

The concentration and administration frequency of the active ingredient of the eye drops used in the present invention may vary according to, for example, the compound to be used, the kind of subjects (such as animals or humans), age, weight, symptoms to be treated, effects of treatment to be desired, administration route, administration volume and period of treatment. Accordingly, suitable concentration and administration frequency may be chosen as desired. Taking an example of isopropyl unoprostone, which is one embodiment of the present invention, one to three drops of the formulation containing 0.01-1.0%, preferably 0.05-0.5%, and for example, 0.12%, 0.15%, 0.18% or 0.3% of isopropyl unoprostone may be administered to an adult 1-10 times a day.

The pharmaceutical composition of the present invention may contain a single active ingredient or a combination of two or more active ingredients. In a combination of plural active ingredients, their respective contents may be suitably increased or decreased in consideration of their therapeutic effects and safety.

Further, the formulations of the present invention may suitably contain the other pharmacologically active ingredients, as far as they are not contrary to the objects of the present invention.

The present invention will be described in detail with reference to the following example, which, however, is not intended to limit the scope of the present invention.

### EXAMPLE

Clinical study was conducted on patients that had been observed to have GA after exudative AMD treatment.

### SUMMARY OF THE CLINICAL STUDY

A prospective, randomized, placebo-controlled study was performed for forty-eight eyes with GA which had not a recurrence of exudative AMD for at least 6 months after receiving the treatment for exudative AMD. The eyes of the treatment group received two drops of 0.15% isopropyl unoprostone (IU) three times a day for 54 weeks. Participants were examined before starting the study (baseline) and at 4, 8, 12, 24, 36, and 54 weeks to measure GA area, the change in GA, the GA enlargement rate and the logarithm of the minimum angle of resolution (logMAR) visual acuity. The placebo group eyes received the vehicle 3 times a day.

### 1. PARTICIPANTS

All participants met all the following two criteria:
- had been diagnosed with exudative AMD, received the treatment for eliminating the exudative lesions and maintained a dry macula at least six months after the treatment; and
- had detectable geographic atrophy on fundus autofluorescence imaging.

1) Between 50 and 85 years of age (mean 74.7±8.1 years)
2) Sex: Male 30 eyes, Female 18 eyes
   Fifty-two subjects (fifty-two eyes) had been enrolled. However, forty-eight eyes were included in the analyses because four eyes withdrew from the study. The treatment group and the placebo group included 24 eyes each.
3) Subtypes of exudative AMD
   Typical AMD (t-AMD): 29 eyes.
   Polypoidal choroidal vasculopathy: 10 eyes.
   Retinal vascular proliferation: 9 eyes.
4) Treatment History
   Participants had received either one or both of the following treatments for exudative AMD:
   Intravitreal Ranibizumab Injection (IVR; Lucentis^{R} 2.3 mg/0.23 mL was intraviterally injected); and
   Photodynamic therapy (PDT).
5) Informed consent by the participants
   Written informed consent was obtained from each participant who was given with a document for explanation approved by the Institutional Review Board/Ethics Committee of the institution where the clinical study was conducted, and full oral and written explanation about the study.

### 2. TEST DRUG

General name: Isopropyl unoprostone (IU)
Content: 1.5 mg per 1 mL
Manufacturer: R-tech Ueno, Ltd. Tokyo, Japan

### 3. STUDY DESIGN

A prospective randomized, placebo-controlled study was performed. Comparisons were made between the baseline and the value after the treatment. Comparisons were made between the treatment and placebo groups. An alternate assignment scheme was used for randomization.

### 4. DOSAGE AND ADMINISTRATION, DOSING PERIOD AND DOSE OF THE TEST DRUG

Two drops of the test drug or the placebo (vehicle) were administered at 5 minute intervals to the eyes three times a day, i.e. morning, afternoon, and night.
Study duration: 54 weeks.

In the description below, test group will be indicated "U group" and the placebo group will be indicated "P group".

When participants had a recurrence of exudative AMD during the study period, they received the standardized AMD treatment, i.e. IVR therapy, while the administration of the test/placebo eye drops to them was continued. Thus, the participants were not regarded as a dropout.

### 5. EVALUATION

The participants were examined to measure the following items at enrollment (baseline) and at 4, 8, 12, 24, 36 and 54 weeks.

### 1) GA area

Fundus autofluorescence images were obtained using BluePeak™ blue laser fundus autofluorescence (FAF) and confocal scanning laser ophthalmoscopy reflectance (CSLO). Hypofluorescent areas that reflect decreased autofluorescence were defined as GA areas. In detail, digital images obtained as above were calibrated and analyzed using RegionFinder™ software (version 2.4, Heidelberg Engineering, Germany) to define and quantify GA areas. Obtaining the fundus autofluorescence images, analyzing the obtained images and defining GA areas were performed according to the manuals of the equipment and software.

At each measurement time, the GA size, the change in GA area from the baseline and the GA enlargement rate from the baseline were determined.

### 2) Visual acuity

Visual acuity was examined and the obtained value was converted in the logarithm of the minimum angle of resolution (LogMar) to give logMar visual acuity.

### 3) Others

Other tests, including objective tests e.g., slit-lamp biomicroscopy, intraocular pressure, fundus examination, MAIA fine visual field inspection (central 10-2), and optical coherence tomography (OCT) test were also carried out (data not shown).

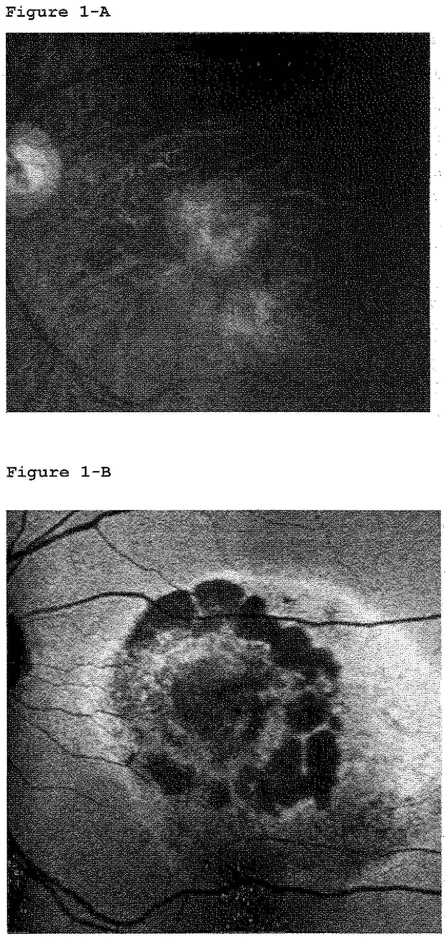

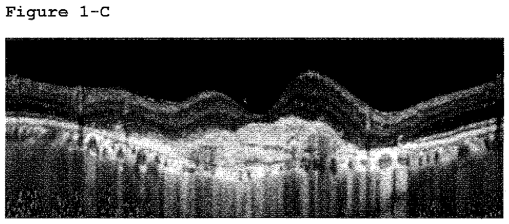

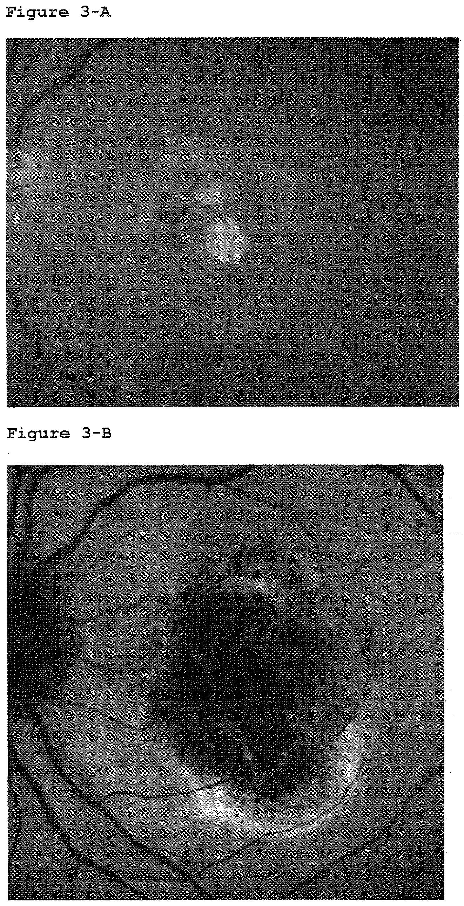

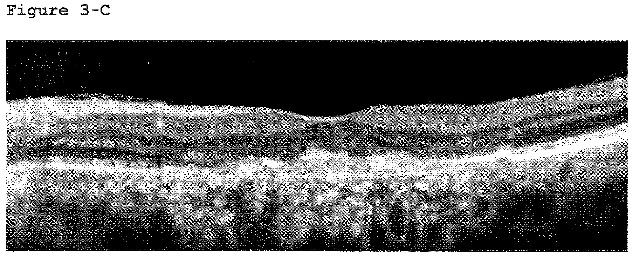

Representative cases are shown below.
U group: The left eye of a 74 years old man with typical AMD. He had received 3 prior PDT treatments and 10 prior IVR injections (Figure 1).

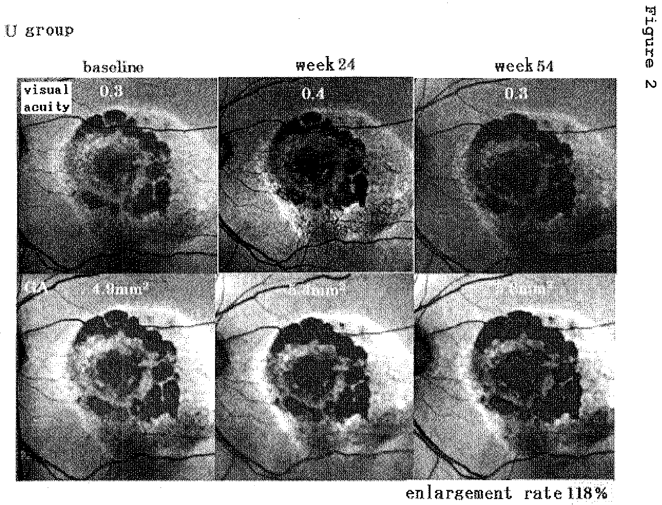

   Figures 1-A, 1-B and 1-C show fundus photograph, fundus autofluorescence image and optical coherence tomography image, at baseline, respectively. Figure 2 shows fundus autofluorescence images at baseline, 24 weeks, and 54 weeks as well as images with GA areas defined by using RegionFinder. GA areas are shown by solid shade. The GA enlargement rate at 54 week was 118%.
P group: The left eye of a 79 years old woman with typical AMD. She had received 1 prior PDT treatment and 18 prior IVR injections (Figure 3).

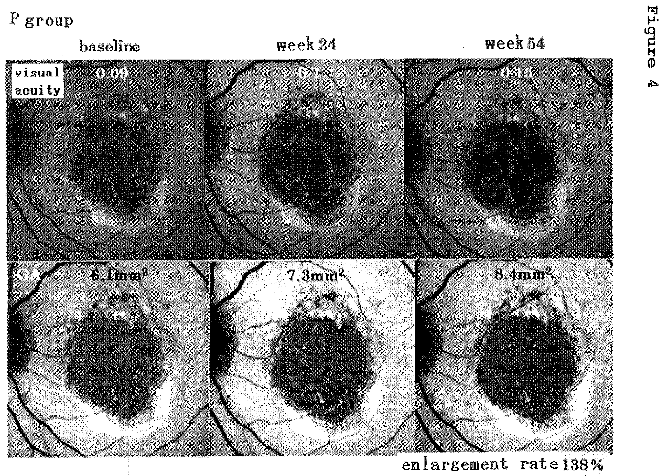

   Figures 3-A, 3-B and 3-C show fundus photograph, fundus autofluorescence image and optical coherence tomography image, at baseline, respectively. Figure 4 shows fundus autofluorescence images at baseline, 24 weeks, and 54 weeks as well as images with GA areas defined by using RegionFinder. GA areas are shown by solid shade. The GA enlargement rate at 54 week was 138%.

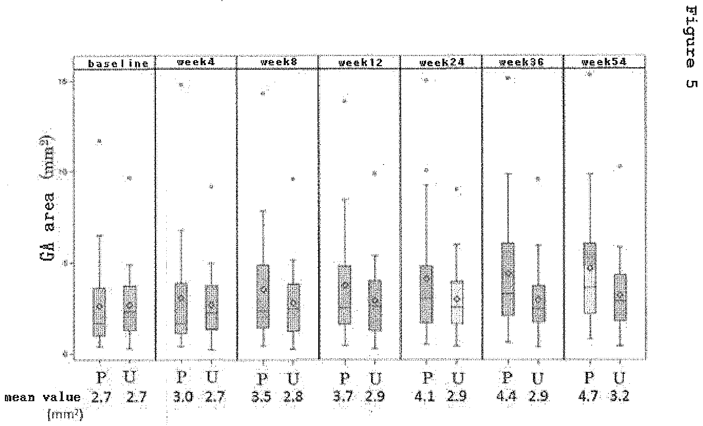

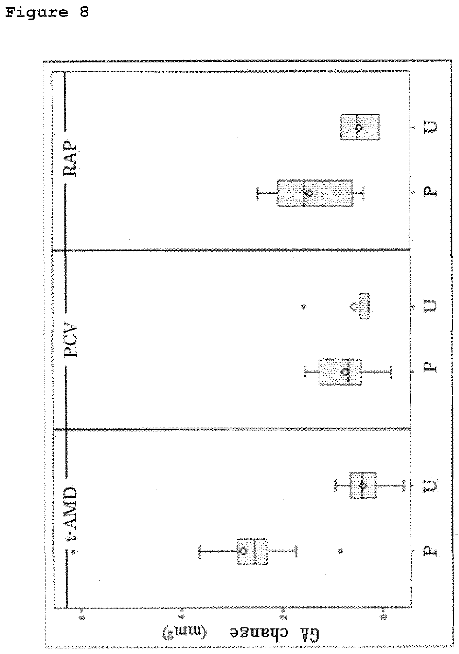

The results are summarized in Table 1 below and Figures 5-8.

**Table 1**

| Background factors | Category | U group | | P group | | Total | |
|---|---|---|---|---|---|---|---|
| Number of Subjects | | 24 | | 24 | | 48 | |
| Age (years) | mean±SD | 74.0±8.5 | | 75.3±7.9 | | 74.7+8.1 | |
| | range | 50 to 85 | | 61 to 85 | | 50 to 5-6, | |
| Sex | Male | 16 | (66.7%) | 14 | (58.3%) | 30 | (62.5%) |
| | Female | 8 | (33.3%) | 10 | (41.7%) | 18 | (37.5%) |
| AMD Subtype | AMD | 16 | (66.7%) | 13 | (54.2%) | 29 | 5-6, |
| | PCV | 5 | (20.8%) | 5 | (20.8%) | 10 | (20.8%) |
| | RAP | 3 | (12.5%) | 6 | (25.0%) | 9 | (18.8%) |
| Recurrence | yes | 6 | (25.0%) | 7 | (29.2%) | 13 | (27.1%) |
| | No | 18 | (75.0%) | 17 | (70.8%) | 35 | (72.9%) |
| Number of prior treatments | IVR | 5.0±3.5 | | 4.7±4.1 | | 4.9±3.7 | |
| | PDT | 1.2±1.8 | | 1.2±1.3 | | 1.2±1.6 | |

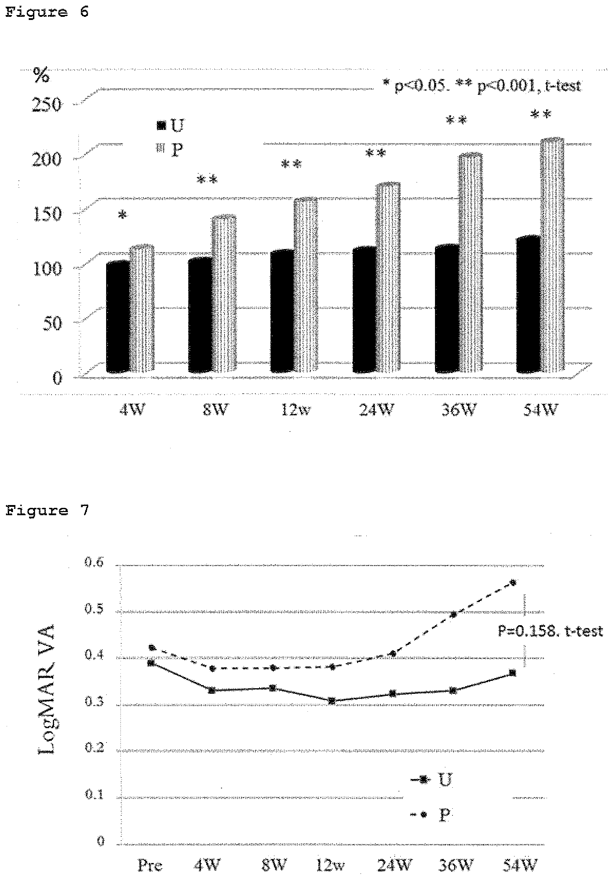

Mean GA areas before enrollment (baseline) were about 2.7 mm² in both U and P groups (U group: 2.71 ± 2.03 mm²; P group: 2.65 ± 2.60 mm²). There was no statistically significant difference in GA area between the U and P groups throughout the study. On the other hand, the difference of changes in GA area from the baseline were statistically significant between the U and P groups (p<0.05 at week 4, and p<0.001 at weeks 8 to 54 (t-test)). The mean change in GA area at week 54 in the U and P groups were 0.44 ± 0.39 mm² and 2.01 ± 1.34mm², respectively (Figure 5). The GA enlargement rate between U and P groups were also significantly different (p <0.05 at week 4, p <0.001 at weeks 8 to 54 (t-test)). The GA enlargement ratio at week 54 in the U and P groups were 1.2 ± 0.2 times (120.8 ± 15.0%) and 2.1 ± 1.0 times (210.8 ± 95.7%), respectively (Figure 6). From these results, it was concluded that isopropyl unoprostone (IU) eye drops could suppress the enlargement of GA.

The time-dependent change in the logMAR visual acuity is shown in Figure 7. Baseline visual acuities in the U and P groups were 0.39 ± 0.39 and 0.42 ± 0.36, respectively. Visual acuities in the U and P groups were 0.33 ± 0.34 and 0.38 ± 0.33 (p = 0.62, t-test) at week 4, and 0.37 ± 0.34 and 0.49 ± 0.39 (p = 0.27, t-test) at week 54, respectively. There was no statistically significant difference in logMAR visual acuity over the baseline. However, logMAR visual acuity in the U group tended to improve over time.

Isopropyl unoprostone (IU) eye drops provided statistically significant suppression of the change in geographic atrophy (GA) area in patients who had received the treatment for exudative AMD and developed geographic atrophy (GA) after the treatment.

## Claims

1. A pharmaceutical composition for use in the treatment of geographic atrophy associated with age-related macular degeneration, which comprises a 15-keto-prostaglandin compound.

2. The composition as described in Claim 1, wherein said 15-keto-prostaglandin compound is a compound of formula (I). 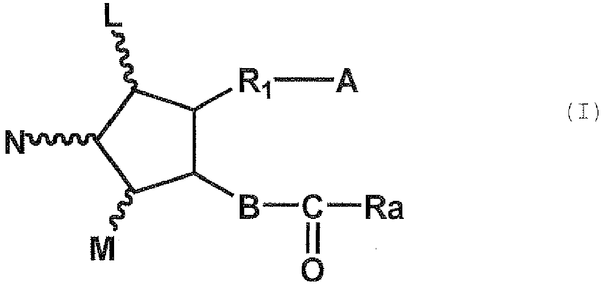
wherein L, M and N are hydrogen, hydroxy, halogen, lower alkyl, hydroxy(lower)alkyl, or oxo, and the five-membered ring may have at least one double bond;
A is -CH₃, -CH₂OH, -COCH₂OH, -COOH or a functional derivative thereof;
B is -CH₂-CH₂-, -CH=CH- or -C=C-;
R₁ is a saturated or unsaturated bivalent lower or medium aliphatic hydrocarbon residue, which is unsubstituted or substituted with halogen, lower alkyl, hydroxy, oxo, aryl or heterocyclic group, and at least one of carbon atom in the aliphatic hydrocarbon is optionally substituted by oxygen, nitrogen or sulfur; and
Ra is a saturated or unsaturated lower or medium aliphatic hydrocarbon group, which is unsubstituted or substituted with halogen, oxo, hydroxy, lower alkyl, lower alkoxy, lower alkanoyloxy, cyclo(lower)alkyl, cyclo(lower)alkyloxy, aryl, aryloxy, heterocyclic group or hetrocyclic-oxy group; lower alkoxy, lower aklanoyloxy, cyclo(lower)alkyl; cyclo(lower)alkyloxy; aryl; aryloxy; heterocyclic group; heterocyclic-oxy group.

3. The composition as described in Claim 1, wherein said 15-keto-prostaglandin compound is a 13,14-dihydro-15-keto- prostaglandin compound.

4. The composition as described in Claim 1, wherein said 15-keto-prostaglandin compound is a 15-keto-20-lower alkyl-prostaglandin compound.

5. The composition as described in Claim 1, wherein said 15-keto-prostaglandin compound is a 13,14-dihydro-15-keto-20-lower alkyl-prostaglandin compound.

6. The composition as described in Claim 1, wherein said 15-keto-prostaglandin compound is a 15-keto-20-ethyl-prostaglandin F compound.

7. The composition as described in Claim 1, wherein said 15-keto-prostaglandin compound is a 13,14-dihydro-15-keto-20-ethyl-prostaglandin F compound.

8. The composition as described in Claim 1, wherein said 15-keto-prostaglandin compound is a 13,14-dihydro-15-keto- 20-ethyl-prostaglandin F_{2α} compound

9. The composition as described in Claim 1, wherein said 15-keto-prostaglandin compound is a 13,14-dihydro-15-keto- 20-ethyl-prostaglandin F_{2α} isopropyl ester.

10. The composition as described in any one of Claims 1-9, wherein said age related macular degeneration is exudative age related macular degeneration.

11. The composition as described in Claim 10, wherein the composition is for treating geographic atrophy in a subject whose exudative lesion has disappeared by the conventional treatment for exudative macular.

12. The composition as described in any one of Claims 1-9, wherein said age related macular degeneration is dry age related macular degeneration.

13. The composition as described in any one of Claims 1-12, wherein said composition is formulated as a composition for topical administration.

14. The composition as described in Claim 13, wherein said composition is an ophthalmic composition for ocular topical administration.

15. The composition as described in Claim 14, wherein said ophthalmic composition is formulated as eye drops.

16. The composition as described in Claim 15, wherein said eye drop is formulated as a sterile unit dose type eye drop containing no preservatives.

17. The composition as described in Claim 14 or 15, wherein said ophthalmic composition comprises substantially no benzalkonium chloride.
